# EUROPÄISCHE PATENTANMELDUNG

(11) **EP 1 389 608 A1**
(43) Veröffentlichungstag der Anmeldung: **18.02.2004**
(21) Anmeldenummer: 03016678.9
(22) Anmeldetag: 01.08.2003
(51) Int. Cl.: C07C 45/44

(54) **Katalytische Reduktion von Benzonitrilderivaten zu Benzaldehydderivaten**

(30) Priorität: 14.08.2002 DE 10237196
(71) Anmelder: Bayer Chemicals AG, 51368 Leverkusen (DE)
(72) Erfinder: Eckert, Markus, Dr., Shanghai 200001 (CN); Dürholz, Friedrich, Dr., 42897 Remscheid (DE)

(57) **Zusammenfassung**

Die Erfindung betrifft ein Verfahren zur katalytischen Reduktion von substituierten Benzonitrilen zu substituierten Benzaldehyden in Gegenwart wäßriger Säuren, eines Nickel- und Aluminium- enthaltenden Katalysators und Wasserstoff.

## Beschreibung

Die Erfindung betrifft ein Verfahren zur katalytischen Reduktion von substituierten Benzonitrilen zu substituierten Benzaldehyden in Gegenwart von Wasser, Säure, eines Nickel- und Aluminium- enthaltenden Katalysators und Wasserstoff.

Substituierte Benzaldehyde besitzen als Feinchemikalien und Wirkstoffzwischenprodukte eine große industrielle Bedeutung.

Die Darstellung von substituierten Benzaldehyden erfolgt häufig durch die selektive Reduktion von den entsprechenden Benzoesäurederivaten, meist mit komplexen Metallhydriden oder mit einem Palladiumkatalysator nach der Methode von Rosenmund. Diese Verfahren haben den Nachteil, dass sie entweder teure Hydridquellen oder teure Katalysatoren verwenden. Zudem werden die eingesetzten Benzoesäurederivate meist aus den entsprechenden Benzonitrilen durch Verseifung hergestellt, so dass es von Vorteil wäre, direkt das Benzonitril zum Benzaldehyd zu konvertieren.

In US 5,124,487 wird beschrieben, dass p-Trifluormethylbenzaldehyde aus den p-Trifluormethylbenzonitrilen durch eine katalytische Reduktion mit Wasserstoff in wässriger Ameisensäure in Gegenwart eines Nickel/Aluminium Katalysators hergestellt werden können. Das Verfahren ist jedoch auf spezielle Substitutionsmuster und insbesondere auf das Vorhandensein einer stark elektronenziehenden, para-ständigen Trifluormethylgruppe beschränkt.

Überraschend wurde nun ein Verfahren zur Herstellung von Benzaldehyden der Formel (I) gefunden, 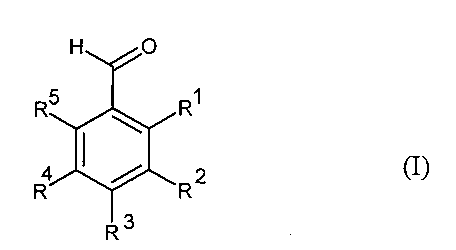 in der
- R¹, R², R⁴ und R⁵: jeweils unabhängig voneinander stehen für: Wasserstoff, Fluor, freies oder geschütztes Formyl, C₁-C₁₂-Alkyl, C₁-C₁₂-Alkoxy, C₁-C₁₂-Halogenalkyl, C₁-C₁₂-Halogenalkoxy, C₄-C₁₄-Aryl, C₅-C₁₅-Arylalkyl, -PO-[(C₁-C₈)-Alkyl]₂, -PO-[(C₄-C₁₄)-Aryl]₂, -PO-[(C₁-C₈)-Alkyl)(C₄-C₁₄)-Aryl)], Tri(C₁-C₈-alkyl)siloxyl
oder für Reste der Formel (II),

A-CO-B (II)

in der unabhängig voneinander
- A: fehlt oder für einen C₁-C₈-Alkylenrest steht und
- B: für R⁶, OR⁶, NHR⁷ oder N(R⁷)₂ steht,
wobei R⁶ für C₁-C₈-Alkyl, C₅-C₁₅-Arylalkyl, C₁-C₈-Halogenalkyl oder C₄-C₁₄-Aryl und
R⁷ jeweils unabhängig für C₁-C₈-Alkyl, C₅-C₁₅-Arylalkyl oder C₅-C₁₄-Aryl oder N(R⁷)₂ zusammen für einen cyclischen Aminorest steht,
oder für Reste der Formeln (IIIa-e)

A-E (IIIa)

A-SO₂-B (IIIb)

A-SO₂R⁶ (IIIc)

A-SO₃W (IIId)

A-COW (IIIe)

in denen
- A, B und R⁶: die oben angegebene Bedeutung besitzen und
- W: für OH oder NH₂ steht,
und
- R³: für Wasserstoff, Fluor, Chlor oder Brom steht,
das dadurch gekennzeichnet ist, dass Verbindungen der Formel (IV) 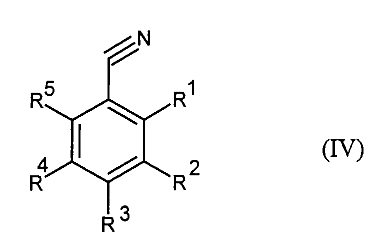 in der
R¹, R², R³, R⁴ und R⁵ die unter der Formel (I) genannte Bedeutung besitzen
- in Gegenwart von Wasser und
- in Gegenwart von Säure oder sauren Salzen, wobei die Säuren oder die sauren Salze bezogen auf ein wässriges Bezugssystem bei 25°C einem pKₛ-Wert von 1 bis 6 aufweisen und
- in Gegenwart eines Nickel und Aluminium enthaltenden Katalysators
- mit Wasserstoff
umgesetzt werden.

**Alkyl** beziehungsweise **Alkoxy** bedeutet jeweils unabhängig einen geradkettigen, cyclischen, verzweigten oder unverzweigten Alkyl- beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, neo-Pentyl, cyclo-Hexyl, cyclo-Pentyl, n-Hexyl, n-Heptyl und n-Octyl, C₁-C₁₂-Alkyl weiter darüber hinaus beispielsweise für n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüber hinaus für n-Pentoxy, cyclo-Hexoxy, cyclo-Pentoxy, n-Hexoxy und n-Octoxy, C₁-C₁₂-Alkoxy weiter darüber hinaus beispielsweise für n-Decoxy und n-Dodecoxy.

**Halogenalkyl** beziehungsweise **Halogenalkoxy** bedeutet jeweils unabhängig einen geradkettigen, cyclischen, verzweigten oder unverzweigten Alkyl-Rest bzw. Alkoxy-Rest, der einfach, mehrfach oder vollständig durch Brom, Chlor und/oder Fluoratome, bevorzugt durch Fluoratome substituiert ist.

Beispielsweise steht C₁-C₁₂-Halogenalkyl in allen Zusammenhängen bevorzugt für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Halogenalkoxy in allen Zusammenhängen bevorzugt für Trifluormethoxy, 2,2,2-Trifluorethoxy, Heptafluorisopropoxy und Pentafluorethoxy.

**Aryl** bedeutet jeweils unabhängig einen heteroaromatischen Rest mit 4 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Beispiele für carbocyclische aromatische Reste mit 6 bis 14 Gerüstkohlenstoffatomen sind zum Beispiel Phenyl und Naphtyl, heteroaromatische Reste mit 4 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Alkoxy, C₁-C₁₂-Halogenalkyl, C₁-C₁₂-Halogenalkoxy, Di(C₁-C₈-alkyl)amino, COO(C₁-C₈-Alkyl), CON(C₁-C₈-Alkyl)₂, COO(C₁-C₈-Alkyl), COO(C₄-C₁₄-Aryl), CO(C₁-C₈-Alkyl), C₅-C₁₅-Arylalkyl oder Tri(C₁-C₆-alkyl)siloxyl.

C₄-C₁₄-Aryl steht beispielsweise und bevorzugt für Phenyl, o-, p-, m-Tolyl, o-, p-, m-Anisyl, o-, p-, m-Fluorphenyl, o-, p-, m-Chlorphenyl, o-, p-, m-Trifluormethylphenyl, o-, p-, m-Nitrophenyl und 2-, 3- und 4-Pyridyl.

**Arylalkyl** bedeutet jeweils unabhängig einen geradkettigen, cyclischen, verzweigten oder unverzweigten Alkyl-Rest nach obiger Definition, der einfach, mehrfach oder vollständig durch Aryl-Reste gemäß obiger Definition substituiert sein kann.

C₅-C₁₅-Arylalkyl steht beispielsweise und bevorzugt für Benzyl oder (R)- oder (S)-1-Phenylethyl.

**Geschütztes Formyl** bedeutet einen Formyl-Rest, der durch Überrührung in ein Aminal, Acetal oder ein gemischtes Aminalacetal geschützt ist, wobei die Aminale, Acetale und gemischten Aminalacetale acyclisch oder cyclisch sein können.

Beispielsweise und bevorzugt steht geschütztes Formyl für einen 1,1-(2,5-Dioxy)-cyclopentylen-Rest.

Im Folgenden werden die bevorzugten Substitutionsmuster für Verbindungen der Formeln (I) und (II) definiert:
R¹, R², R⁴ und R⁵ stehen bevorzugt jeweils unabhängig voneinander für Wasserstoff, Fluor, C₁-C₄-Alkyl, C₁-C₄-Alkoxy, C₁-C₄-Halogenalkyl, C₁-C₄-Halogenalkoxy oder N(R⁷)₂, wobei R⁷ jeweils unabhängig bevorzugt jedoch identisch für Methyl oder Ethyl stehen.
R¹, R², R⁴ und R⁵ stehen besonders bevorzugt jeweils unabhängig voneinander für Wasserstoff, Fluor, Trifluormethyl und Pentafluorethyl, ganz besonders bevorzugt für Wasserstoff oder Trifluormethyl.
R³ steht bevorzugt für Wasserstoff, Fluor oder Chlor.

Besonders bevorzugte Verbindungen der Formel (IV) sind: 4-Chlorbenzonitril, 3-Trifluormethylbenzonitril, 3,5-Bis(trifluormethyl)benzonitril und 3,5-Difluorbenzonitril.

Das erfindungsgemäße Verfahren wird in Gegenwart von Wasser und in Gegenwart von Säure oder sauren Salzen durchgeführt, wobei die Säuren oder die sauren Salze bezogen auf ein wässriges Bezugssystem bei 25°C einen pK_{S}-Wert von 1 bis 6 aufweisen. Bevorzugt beträgt das Gewichtsverhältnis von Säure und/oder saurem Salz zu Wasser 1:10 bis 10:1, besonders bevorzugt 1:1 bis 4:1.

Bevorzugte Säuren sind Carbonsäuren wie insbesondere Ameisensäure, Essigsäure oder Propionsäure, wobei Ameisensäure und Essigsäure weiter bevorzugt sind.

Das erfindungsgemäße Verfahren wird in Gegenwart eines Nickel und Aluminium enthaltenden Katalysators durchgeführt.

Dabei kann das Gewichtsverhältnis von Nickel und Aluminium beispielsweise 1:20 bis 20:1, bevorzugt 1:5 bis 5:1 und besonders bevorzugt 0,9:1 bis 1,1:1 betragen.

Der Einsatz des Nickel und Aluminium enthaltenden Katalysators kann sowohl als Mischung der Metalle oder in Form einer Legierung erfolgen, wobei der Einsatz als Legierung bevorzugt ist.

Vorzugsweise wird der Nickel und Aluminium enthaltende Katalysator in feinverteilter Form beispielsweise als Pulver oder Staub oder in Form von Stücken eingesetzt.

Weiterhin kann der Nickel und Aluminium enthaltende Katalysator ein oder mehrere Metalle aus der Gruppe Chrom, Rhenium, Eisen, Cobalt, Molybdän und Kupfer enthalten.

Die Menge des Nickel und Aluminium enthaltenden Katalysators kann beispielsweise 0,5 bis 50 Gewichtsprozent bezogen auf die eingesetzte Verbindung der Formel (IV) betragen, bevorzugt 3 bis 30 Gewichtsprozent und besonders bevorzugt 5 bis 20 Gewichtsprozent. Größere Mengen an Katalysator sind möglich aber unwirtschaftlich.

Erfindungsgemäß erfolgt die Umsetzung von Verbindungen der Formel (IV) mit Wasserstoff.

Der Wasserstoffdruck kann beispielsweise bei 0,2 bis 100 bar betragen, bevorzugt 1 bis 20 bar, ganz besonders bevorzugt 2 bis 10 bar.

Die Reaktionstemperatur kann zum Beispiel 20°C bis 200°C, bevorzugt 40 bis 120°C und besonders bevorzugt 60°C bis 90°C betragen.

Die Reaktionsdauer kann beispielsweise 0,2 h bis 72 Stunden betragen, bevorzugt 1 bis 36 h und ganz besonders bevorzugt 2 bis 10 h.

In einer bevorzugten Ausführungsform des erfindungsgemäßen Verfahrens legt man Säure oder saures Salz, Wasser, den Katalysator und die Verbindungen der Formel (IV) vor, setzt das Reaktionsgemisch unter Wasserstoffdruck, bevorzugt unter einen Wasserstoffdruck von etwa 1 bar, und erwärmt das Gemisch, beispielsweise in 30 Minuten bis 2 Stunden, auf Reaktionstemperatur. Bei Erreichen der Reaktionstemperatur wird der Wasserstoffdruck auf den gewünschten Wert erhöht und in einer weiter bevorzugten Ausführungsform bis zum vollständigen Umsatz isobar gehalten.

Alternativ dazu kann die Verbindung der Formel (IV) auch zu der Reaktionsmischung zugepumpt werden.

Es ist von Vorteil, bei der Aufarbeitung zunächst den Katalysator abzufiltrieren und die Reaktionslösung anschließend mit einem organischen Lösungsmittel wie beispielsweise Toluol zu extrahieren. Das Extrakt wird vom Lösungsmittel befreit und der Rückstand via Destillation oder Umkristallisation gereinigt.

Auf erfindungsgemäße Weise werden Verbindungen der Formel (I) erhalten.

Das erfindungsgemäße Verfahren eignet sich insbesondere für die Herstellung von 3-Trifluormethylbenzaldehyd, 3,5-Bis(trifluormethyl)benzaldehyd, 4-Chlorbenzaldehyd und 3,5-Difluorbenzaldehyd.

Die erfindungsgemäß hergestellten Verbindungen der Formel (I) können, vorzugsweise ohne Zwischenisolierung, mit Wasserstoff und in Gegenwart eines Katalysators zu den Verbindungen der Formel (V) reduziert werden, wobei in Formel (V)

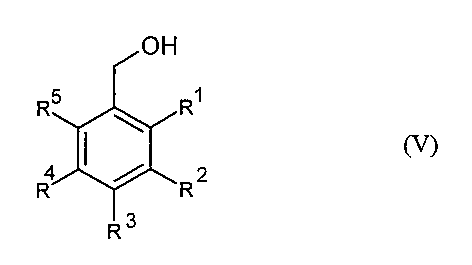

R¹, R², R³, R⁴ und R⁵ die unter der Formel (I) genannten Bedeutungen einschließlich der genannten Vorzugsbereiche besitzen.

Beispielsweise kann dies dadurch geschehen, dass nach der erfindungsgemäßen Darstellung der Verbindungen der Formel (IV) Raney-Nickel zu der Reaktionslösung zugesetzt wird und unter einem Wasserstoffdruck von 5 bis 200 bar, bevorzugt 20 bis 100 bar reduziert wird oder die erfindungsgemäß dargestellten Benzaldehyde zunächst extrahiert werden und die organische Phase anschließend zum Beispiel unter Zusatz von Raney Nickel als Katalysator wie oben beschrieben reduziert werden.

Die erfindungsgemäß herstellbaren Verbindungen der Formeln (IV) und (V) eignen sich insbesondere in einem Verfahren zur Herstellung von Agrochemikalien und Pharmazeutika.

Der Vorteil des erfindungsgemäßen Verfahrens liegt in der leichten Durchführbarkeit und der Einsparung chemischer Verfahrensstufen gegenüber den klassischen Verfahren zur Herstellung von Benzaldehyden aus Benzonitrilen.

### Beispiele

### Beispiel 1:

Es wurden 10,8 g 3,5-Bis(trifluormethyl)benzonitril in 70 g 80 %iger Ameisensäure sowie 0,9 g Alloy Ni-Al-50/50 in einem Autoklaven vorgelegt. Der Autoklav wurde mit 15 bar Stickstoff abgedrückt und auf 70°C geheizt. Bei Erreichen der Reaktionstemperatur wurde der Wasserstoffdruck auf 3 bar erhöht und 10 h isobar gehalten. Anschließend wurde abgekühlt, das Gemisch filtriert, die Lösung mit Toluol extrahiert und anschließend im Vakuum fraktioniert destilliert. Es wurden 8,8 g 3,5-Bis(trifluormethyl)benzaldehyd erhalten (80 % d.Th.).

### Beispiel 2:

Es wurden 7,7 g 3-Trifluormethylbenzonitril in 70 g 70 %iger Ameisensäure sowie 0,9 g Alloy Ni-Al-50/50 in einem Autoklaven vorgelegt. Der Autoklav wurde mit 15 bar Stickstoff abgedrückt und auf 80°C geheizt. Bei Erreichen der Reaktionstemperatur wurde der Wasserstoffdruck auf 10 bar erhöht und 7 h isobar gehalten. Anschließend wurde abgekühlt, das Gemisch filtriert, die Lösung mit Toluol extrahiert und anschließend im Vakuum fraktioniert destilliert. Es wurden 6,5 g 3-Trifluormethylbenzaldehyd erhalten (85 % d.Th.).

### Beispiel 3:

Es wurden 7,7 g 3-Trifluormethylbenzonitril in 50 g 75 %iger Ameisensäure sowie 1 g NiFeCrAl Vorlegierung in einem Autoklaven vorgelegt. Der Autoklav wurde mit 15 bar Stickstoff abgedrückt und auf 80°C geheizt. Bei Erreichen der Reaktionstemperatur wurde der Wasserstoffdruck auf 6 bar erhöht und 10 h isobar gehalten. Anschließend wurde abgekühlt, das Gemisch filtriert, die Lösung mit Toluol extrahiert und anschließend im Vakuum fraktioniert destilliert. Es wurden 7,0 g 3-Triflurirmethylbenzaldehy erhalten (89 % d.Th.).

### Beispiel 4:

Es wurden 6,3 g 3,5-Difluorbenzonitril in 50 g 75 %iger Ameisensäure sowie 0,7 g Alloy Ni-Al-50/50 in einem Autoklaven vorgelegt. Der Autoklav wurde mit 15 bar Stickstoff abgedrückt und auf 70 °C geheizt. Bei Erreichen der Reaktionstemperatur wurde der Wasserstoffdruck auf 3 bar erhöht und 10 h isobar gehalten. Anschließend wurde abgekühlt, das Gemisch filtriert, die Lösung mit Toluol extrahiert und anschließend im Vakuum fraktioniert destilliert. Es wurden 3,9 g 3,5-Difluorbenzaldehyd erhalten (61 % d.Th.).

### Beispiel 5:

Es wurden 16,5 g 4-Chlorbenzonitril in 139 g 75 %iger Ameisensäure sowie 1,4 g Alloy Ni-Al-50/50 in einem Autoklaven vorgelegt. Der Autoklav wurde mit 15 bar Stickstoff abgedrückt und auf 70°C geheizt. Bei Erreichen der Reaktionstemperatur wurde der Wasserstoffdruck auf 3 bar erhöht und 10 h isobar gehalten. Anschließend wurde abgekühlt, das Gemisch filtriert, die Lösung mit Toluol extrahiert und anschließend im Vakuum fraktioniert destilliert. Es wurden 15,1 g 4-Chlorbenzaldehyd erhalten (89 % d.Th.).

### Beispiel 6:

Es wurden 14,5 g 2-Fluorbenzonitril in 122 g 75 %iger Ameisensäure sowie 1,2 g Alloy Ni-Al-50/50 in einem Autoklaven vorgelegt. Der Autoklav wurde mit 15 bar Stickstoff abgedrückt und auf 70°C geheizt. Bei Erreichen der Reaktionstemperatur wurde der Wasserstoffdruck auf 3 bar erhöht und 10 h isobar gehalten. Anschließend wurde abgekühlt, das Gemisch filtriert, die Lösung mit Toluol extrahiert und anschließend im Vakuum fraktioniert destilliert. Es wurden 12,0 g 2-Fluorbenzaldehyd erhalten (80 % d.Th.).

## Patentansprüche

1. Verfahren zur Herstellung von Verbindungen der Formel (I), 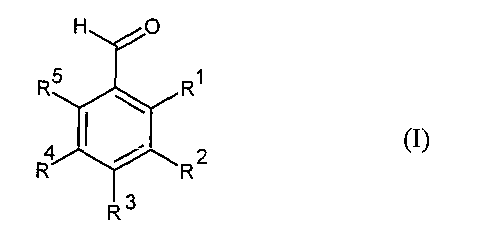 in der
R¹, R², R⁴ und R⁵ jeweils unabhängig voneinander stehen für: Wasserstoff, Fluor, freies oder geschütztes Formyl, C₁-C₁₂-Alkyl, C₁-C₁₂-Alkoxy, C₁-C₁₂-Halogenalkyl, C₁-C₁₂-Halogenalkoxy, C₄-C₁₄-Aryl, C₅-C₁₅-Arylalkyl, -PO-[(C₁-C₈)-Alkyl]₂, -PO-[(C₄-C₁₄)-Aryl]₂, -PO-[(C₁-C₈)-Alkyl)(C₄-C₁₄)-Aryl)], Tri(C₁-C₈-alkyl)siloxyl
oder für Reste der Formel (II),
A-CO-B (II)
in der unabhängig voneinander
A fehlt oder für einen C₁-C₈-Alkylenrest steht und
B für R⁶, OR⁶_{,} NHR⁷ oder N(R⁷)₂ steht,
wobei R⁶ für C₁-C₈-Alkyl, C₅-C₁₅-Arylalkyl, C₁-C₈-Halogenalkyl oder C₄-C₁₄-Aryl und
R⁷ jeweils unabhängig für C₁-C₈-Alkyl, C₅-C₁₅-Arylalkyl oder C₅-C₁₄-Aryl oder N(R⁷)₂ zusammen für einen cyclischen Aminorest steht,
oder für Reste der Formeln (IIIa-e)
A-E (IIIa)
A-SO₂-B (IIIb)
A-SO₂R⁶ (IIIc)
A-SO₃W (IIId)
A-COW (IIIe)
in denen
A, B und R⁶ die oben angegebene Bedeutung besitzen und
W für OH oder NH₂ steht,
und
R³ für Wasserstoff, Fluor, Chlor oder Brom steht,
**dadurch gekennzeichnet, dass** Verbindungen der Formel (IV) 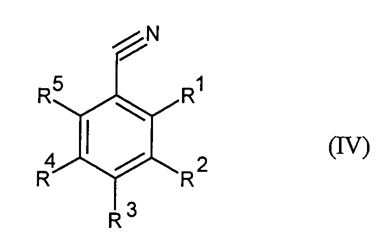 in der
R¹, R², R³, R⁴ und R⁵ die unter der Formel (I) genannte Bedeutung besitzen
• in Gegenwart von Wasser und
• in Gegenwart von Säure oder sauren Salzen, wobei die Säuren oder die sauren Salze bezogen auf ein wässriges Bezugssystem bei 25°C einem pKₛ-Wert von 1 bis 6 aufweisen und
• in Gegenwart eines Nickel und Aluminium enthaltenden Katalysators
• mit Wasserstoff
umgesetzt werden.

2. Verfahren gemäß Anspruch 1, **dadurch gekennzeichnet, dass** R¹, R², R⁴ und R⁵ jeweils unabhängig voneinander für Wasserstoff, Fluor, C₁-C₄-Alkyl, C₁-C₄-Alkoxy, C₁-C₄-Halogenalkyl, C₁-C₄-Halogenalkoxy oder N(R⁷)₂ stehen, wobei R⁷ jeweils unabhängig bevorzugt jedoch identisch für Methyl oder Ethyl stehen.

3. Verfahren gemäß mindestens einem der Ansprüche 1 und 2, **dadurch gekennzeichnet, dass** als Verbindungen der Formel (IV) 4-Chlorbenzonitril, 3-Trifluormethylbenzonitril, 3,5-Bis(trifluormethyl)benzonitril oder 3,5-Difluorbenzonitril eingesetzt werden.

4. Verfahren gemäß mindestens einem der Ansprüche 1 bis 3, **dadurch gekennzeichnet, dass** das Gewichtsverhältnis von Säure und/oder saurem Salz zu Wasser 1:10 bis 10:1 beträgt.

5. Verfahren gemäß mindestens einem der Ansprüche 1 bis 4, **dadurch gekennzeichnet, dass** als Säuren Carbonsäuren verwendet werden.

6. Verfahren gemäß mindestens einem der Ansprüche 1 bis 5, **dadurch gekennzeichnet, dass** der Einsatz des Nickel und Aluminium enthaltenden Katalysators als Mischung der Metalle oder in Form einer Legierung erfolgt.

7. Verfahren gemäß mindestens einem der Ansprüche 1 bis 6, **dadurch gekennzeichnet, dass** der Nickel und Aluminium enthaltende Katalysator ein oder mehrere Metalle aus der Gruppe Chrom, Rhenium, Eisen, Cobalt, Molybdän und Kupfer enthält.

8. Verfahren gemäß mindestens einem der Ansprüche 1 bis 7, **dadurch gekennzeichnet, dass** die Menge des Nickel und Aluminium enthaltenden Katalysators 0,5 bis 50 Gewichtsprozent bezogen auf die eingesetzte Verbindung der Formel (IV) beträgt.

9. Verfahren gemäß mindestens einem der Ansprüche 1 bis 8, **dadurch gekennzeichnet, dass** der Wasserstoffdruck 0,2 bis 100 bar beträgt.

10. Verfahren gemäß mindestens einem der Ansprüche 1 bis 9, **dadurch gekennzeichnet, dass** die Reaktionstemperatur 20°C bis 200°C beträgt.

11. Verfahren gemäß mindestens einem der Ansprüche 1 bis 10, **dadurch gekennzeichnet, dass** Säure oder saures Salz, Wasser, Katalysator und die Verbindung der Formel (IV) vorgelegt werden, das Reaktionsgemisch unter Wasserstoffdruck gesetzt wird und auf Reaktionstemperatur erwärmt wird, bevor der Wasserstoffdruck auf den gewünschten Wert erhöht wird.

12. Verfahren gemäß mindestens einem der Ansprüche 1 bis 11, **dadurch gekennzeichnet, dass** in einem nachfolgenden Schritt die Verbindungen der Formel (I) mit Wasserstoff und in Gegenwart eines Katalysators zu Verbindungen der Formel (V) reduziert werden, wobei in Formel (V) 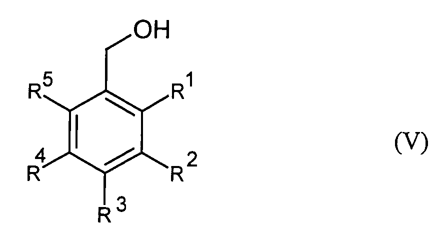 R¹, R², R³, R⁴ und R⁵ die unter der Formel (I) genannten Bedeutungen besitzen.

13. Verfahren gemäß Anspruch 12, **dadurch gekennzeichnet, dass** die Reduktion zu Verbindungen der Formel (V) ohne Zwischenisolierung erfolgt.

14. Verwendung von Verbindungen, die gemäß mindestens einem der Ansprüche 1 bis 13 hergestellt wurden, in einem Verfahren zur Herstellung von Agrochemikalien und Pharmazeutika.
